**Europäisches Patentamt**

**European Patent Office**

**Office européen des brevets**

(11) Veröffentlichungsnummer: **0 017 673**
**B1**

# (12) EUROPÄISCHE PATENTSCHRIFT

(45) Veröffentlichungstag der Patentschrift:
**14.03.84**

(51) Int. Cl.³: **G 01 L 1/08**, G 01 N 33/36

(21) Anmeldenummer: **79105376.2**

(22) Anmeldetag: **27.12.79**

(54) Vorrichtung zur Bestimmung des Querschnitts von Faserbändern.

(30) Priorität: **06.04.79 CH 3290/79**

(43) Veröffentlichungstag der Anmeldung:
**29.10.80 Patentblatt 80/22**

(45) Be anntmachung des Hinweises auf die Patenterteilung:
**14.03.84 Patentblatt 84/11**

(84) Benannte Vertragsstaaten:
**AT BE DE FR GB IT**

(56) Entgegenhaltungen:
**CH - A - 553 981**
**DE - A - 1 959 440**
**DE - A - 2 657 603**
**DE - B - 1 648 798**
**DE - B - 2 031 339**

(73) Patentinhaber: **ZELLWEGER USTER AG, Wilstrasse 11, CH-8610 Uster (CH)**

(72) Erfinder: **Morf, Richard, Stöcklerstrasse 8, CH-8610 Uster (CH)**

(74) Vertreter: **Dipl.-Phys.Dr. Manitz Dipl.-Ing. Finsterwald Dipl.-Ing. Grämkow Dipl.-Chem.Dr. Heyn Dipl.-Phys.Rotermund Morgan B.Sc.(Phys.), Robert-Koch-Strasse 1, D-8000 München 22 (DE)**

Vorrichtung zur Bestimmung des Querschnitts von Faserbändern

Die Erfindung betrifft eine Vorrichtung zur Bestimmung des Querschnitts von Faserbändern, bestehend aus einem eine durchgehende Bohrung aufweisenden Meßtrichter, durch den die Faserbänder unter gleichzeitiger Verdichtung gezogen werden, sowie einer Anordnung zur Ermittlung der für den jeweiligen Faserbandquerschnitt charakteristischen Durchzugskraft.

Die Ermittlung des Querschnitts von Faserbändern zum Zwecke der Gewinnung einer Meßgröße, die zur Regulierung der Faserbanddicke bzw. Bandnummer ausgewertet werden kann, ist in der Praxis von großer Bedeutung.

Es sind auf pneumatischer Basis arbeitende Vorrichtungen zur Gewinnung von Meßgrößen, die vom Substanzquerschnitt von strangförmigem Textilmaterial abhängen, bekannt, wobei sogenannte aktiv-pneumatische und passiv-pneumatische Meßsysteme unterschieden werden.

Aktiv-pneumatische sind solche, bei welchen das Faserband durch einen Trichter hindurchgezogen und die im Faserband eingeschlossene Luft dabei teilweise ausgetrieben wird und einen Druck erzeugt, der an einem seitlich in die Meßdüse mündenden Manometeranschluß entnommen und in ein Meßsignal bzw. Regelsignal umgeformt werden kann. Ein Beispiel einer derartigen Vorrichtung ist in der DE-B-1 648 798 beschrieben.

Als passiv-pneumatische Meßsysteme werden solche bezeichnet, bei welchen in einer Durchflußkammer, welche das Faserband durchläuft, Druckluft zugeführt wird. Dabei werden diejenigen Druckschwankungen als Meßsignal ausgenützt, die dadurch entstehen, daß die Durchflußkammer durch den variablen Faserbandquerschnitt gegen das Ausströmen der zugeführten Druckluft mehr oder weniger stark abgedichtet wird und dadurch Druckschwankungen hervorgerufen werden. Ein derartiges Meßsystem ist in der DE-A1-2 657 603 beschrieben.

Aus der CH-A-553 981 ist eine Vorrichtung zur Messung von Dichteschwankungen eines Faserbandes in Spinnereimaschinen bekannt, welche aus einem Bandtrichter mit sich in Durchlaufrichtung des Faserbandes verengendem Durchlaufkanal und einem Faserbanddichteabweichungen erfassenden Signalgeber besteht, dessen Signal über einen Verstärker weiterverarbeitet wird. Dabei besteht der Bandtrichter aus das durchlaufende Faserband allseitig verdichtenden, radial federnd auslenkbaren, zentrisch symmetrisch angeordneten Gliedern, denen ein oder mehrere Signalgeber hoher Empfindlichkeit zugeordnet sind, von denen jeder ein der Auslenkung entsprechendes Signal an den Verstärker abgibt.

Während sowohl die aktiv-pneumatischen als auch die passiv-pneumatischen Meßsysteme durchwegs Ergebnisse liefern, die nur für Fasermaterial mindestens angenähert konstanter Faserfeinheit brauchbare Ergebnisse liefern, dagegen für Fasermaterial mit stark streuender Faserfeinheit unzuverlässig ist, konnte auch das Meßsystem mit elastisch pendelnd gelagertem Verdichter sich in der Praxis nicht durchsetzen. Gründe hierfür sind wohl in mechanischen Schwierigkeiten zu suchen, indem neben großer Masse des Verdichters, einer aufwendigen Mechanik auch Schwierigkeiten in bezug auf den Ein- und Durchlauf des Faserbandes im Verdichter eine weitere Verbreitung verhinderten. Als solche kommen die Auswirkungen eines nicht ruhig einlaufenden Vlieses, statische Aufladungen oder Veränderungen des Durchzugswiderstandes des Verdichters durch Staub- oder Wachsablagerungen in Frage.

Aufgabe der Erfindung ist es, die vorstehend angeführten Nachteile zu vermeiden und eine sich durch Genauigkeit und hohe Zuverlässigkeit auszeichnende Vorrichtung zur Bestimmung des Querschnitts von Faserbändern zu schaffen.

Die gestellte Aufgabe wird ausgehend von der eingangs definierten Vorrichtung dadurch gelöst, daß der Meßtrichter aus einem festen Teil und einem beweglichen Teil in Form eines Kolbens besteht, durch den sich die Bohrung fortsetzt, wobei auf den beweglichen Teil eine Kraft ausgeübt wird, die der durch die Reibung der Faserbänder in der Bohrung erzeugten Mitnahmetendenz entgegenwirkt und die vom Querschnitt der Faserbänder abhängig ist, und daß eine Einrichtung zur Messung der Relativverschiebung zwischen dem festen und dem beweglichen Teil vorgesehen ist.

Die erfindungsgemäße Vorrichtung bietet eine Anzahl von Vorteilen gegenüber herkömmlichen Systemen.

Ein derartiger Vorteil resultiert daraus, daß der Meßtrichter aus einem festen und einem beweglichen Teil besteht, wobei der feste Teil zunächst eine Vorverdichtung des Faserbandes bewirkt und insbesondere die Faserführung übernimmt. Die Vorverdichtung gestattet es auch, im beweglichen Teil ohne zusätzliche Einschnürung des Durchzugskanals zu arbeiten, da die sich zwischen Faserband und Innenwandung des beweglichen Teils ergebende Reibung ausreicht, um eine vom Querschnitt des Faserbandes abhängige Verschiebung des beweglichen Teils zu bewirken, die dann mit entsprechenden Meßeinrichtungen erfaßt werden kann.

Von Bedeutung ist auch, daß der Meßtrichter lageunabhängig arbeitet, d. h. daß sein Einbau in Produktionsmaschinen nicht durch spezielle Forderungen hinsichtlich einer bestimmten Montagelage beschränkt ist.

Ferner kann die für den Ausgleich der Durchzugskraft zugeführte Druckluft gleichzeitig zur automatischen Reinigung der sich gegeneinander verlagernden Teile und der Hohlräume benutzt werden.

Die Sollwerteinstellung für die mittlere Bandnummer kann ebenfalls durch Regulierung der Druckluftzuführung erfolgen. Dieser Ausgleich kann aber auch mittels einer elektromagnetischen Anordnung unter Verwendung von Spulen und Weicheisenmagneten erreicht werden. Die Bestimmung der Durchzugskraft kann auf pneumatische Weise oder durch elektrische Lageabtastung des beweglichen Teils erfolgen.

Nachfolgend werden Ausführungsbeispiele der Erfindung unter Bezugnahme auf die Zeichnung näher erläutert; in der Zeichnung zeigt

Fig. 1 einen Meßtrichter mit magneto-elektrischem Positionsfühler im Schnitt;

Fig. 2 einen Meßtrichter mit elektromagnetischem Kolbenantrieb und induktiver Stellungsanzeige;

Fig. 3 eine weitere Variante des Meßtrichters gemäß Fig. 1 mit pneumatischem Positionsfühler;

Fig. 4 schematisch den Meßtrichter in einem Regelkreis zur Vergleichmäßigung des Faserbandes;

Fig. 5 schematisch den Meßtrichter als Meßorgan für den Faserbandquerschnitt.

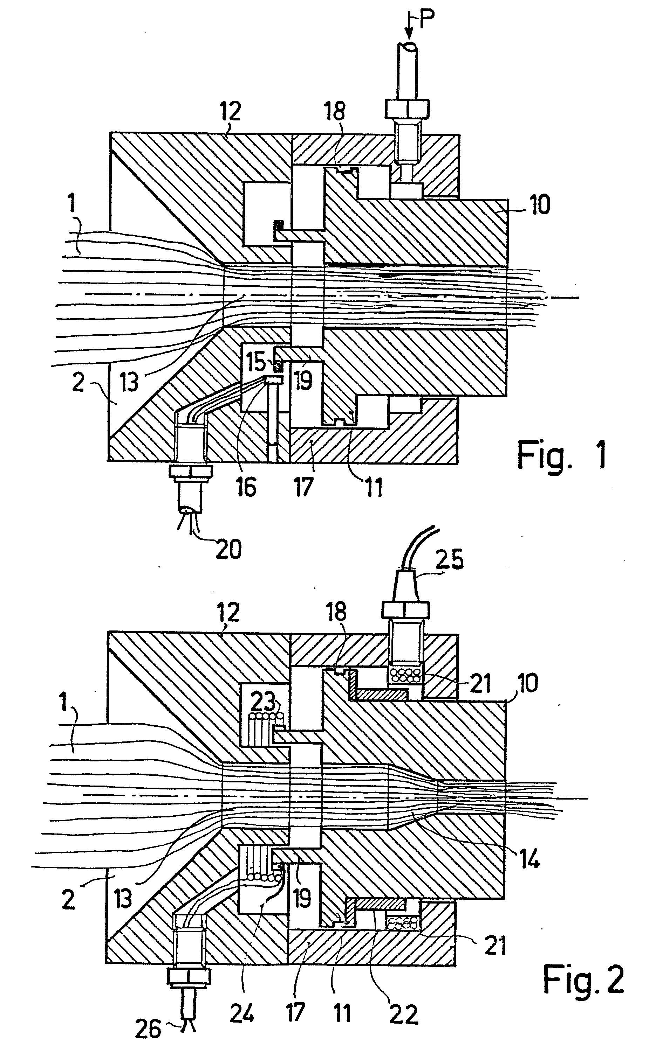

Der Meßtrichter gemäß Fig. 1 besteht aus einem festen Teil 12, der auf der Einlaufseite für das Faserband 1 eine trichterförmige Einlauföffnung 2 aufweist. In dieser wird das Faserband auf den Durchmesser einer durchgehenden Bohrung 13 verdichtet. Am feststehenden Teil 12 ist ein Zylinder 17 aufgeschraubt, in dem ein beweglicher Teil 10, der als Kolben 11 ausgebildet ist, verschiebbar eingesetzt ist. Zwischen dem Kolben 11 und der Innenwand des Zylinders 17 kann ein Luftspalt 18 vorgesehen sein.

Die Bohrung 13 des feststehenden Teils 12 setzt sich im beweglichen Teil 10 fort, wobei der gleiche Durchmesser beibehalten wird. Durch die Reibung zwischen dem Faserband und der Bohrungswand wird beim Durchlauf des Faserbandes eine nach rechts gerichtete Zugkraft auf den beweglichen Teil 10 ausgeübt, deren Größe vom Querschnitt des einlaufenden Faserbandes abhängt.

Um den Kolben 11 bzw. den beweglichen Teil 10 in einer Gleichgewichtslage zu halten, wird in der Anordnung gemäß Fig. 1 in den Zylinder 17 hinter den Kolben 11 Druckluft zugeführt, die den Kolben 11 nach links drängt. Bei geeigneter Bemessung des Druckes P und des Luftspaltes 18 liegt die Mittellage des Kolbens 11 etwa in der Mitte des Zylinders 17. Bei dicker werdendem Band verlagert er sich nach rechts, da die Durchzugskräfte größer werden. Dünneres Band erzeugt weniger Durchzugskraft, so daß der auf den Kolben ausgeübte Druck diesen nach links verlagert.

Es besteht nun die Aufgabe, diese Verlagerung des Kolbens 11 bzw. des beweglichen Teils 10 zu messen bzw. in ein proportionales elektrisches Signal umzuformen. In der Ausführungsform der Fig. 1 wird dies mit einer Feldplatte 16 erreicht. Diese ist im Einflußbereich eines Weicheisenteils 15 angeordnet. Der Weicheisenteil 15 ist auf einer am beweglichen Teil 10 hervorragenden Hülse 19 befestigt. Auf diese Weise werden die Verschiebungen des Weicheisenteils 15 gegenüber der Feldplatte 16 in elektrische Fehlersignale umgeformt, die über Leitungen 20 an entsprechende Meß- oder Regelorgane nach außen geführt sind. Durch Wahl des Druckes P läßt sich der Sollwert des Faserbandquerschnittes einstellen.

Der Meßtrichter nach Fig. 2 unterscheidet sich von der Ausführungsform nach Fig. 1 in seinem Aufbau dadurch, daß die Bohrung im beweglichen Teil 10 sich in ihrem Durchmesser verjüngt, wodurch das Faserband noch weiter zusammengedrückt wird.

Für die Erzeugung der Rückstellkraft ist eine elektromagnetische Anordnung vorgesehen. Es erfolgt dabei eine induktive Lagebestimmung des beweglichen Teils 10 gegenüber dem feststehenden Teil 12.

Einer Induktionsspule 21 wird über Zuleitungen 25 ein Erregerstrom zugeführt. Dadurch erfährt ein Tauchmagnet 22, der um den beweglichen Teil 10 gelegt ist, eine Abstoßung, welche den Kolben 11 nach links — entgegen der Durchzugskraft — drängt. Durch die Größe des Erregerstromes bestimmt sich die Größe der Abstoßung bzw. der aufzuhebenden Durchzugskraft.

Die Messung der Kolbenlage wird mittels eines auf der Hülse 19 angebrachten Weicheisenrings 24 erreicht, der sich innerhalb einer Tauchspule 23 bewegt und deren induktiven Widerstand beeinflußt. Das entsprechende Signal kann an den Signalklemmen 26 entnommen werden.

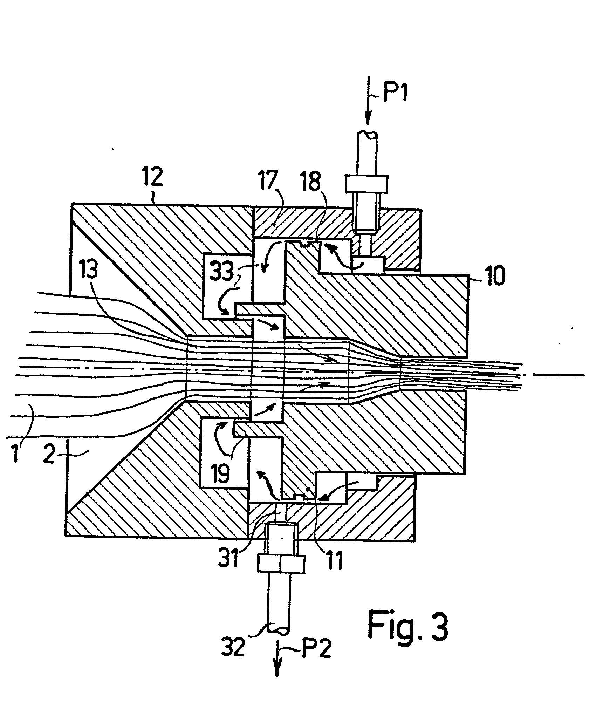

In Fig. 3 ist als weiteres Ausführungsbeispiel eine pneumatische Lagebestimmung des Kolbens 11 innerhalb des Zylinders 17 gezeigt.

Hierzu ist in der Wandung des Zylinders 17 mindestens eine radiale Bohrung 31 vorgesehen, die vom Kolben 11 mindestens teilweise abgedeckt wird. Je nach der Lage des Kolbens 11 wird die Bohrung mehr oder weniger verschlossen. Dadurch entstehen an einer Luftaustrittsöffnung 32 entsprechende Druckwerte P2, die eine Anzeige für die Lage des Kolbens 11 im Zylinder 17 bilden. Die Größe der zugeführten Druckluft P1 stellt wieder ein Maß für den Sollwert des Faserbandquerschnittes dar.

In Fig. 3 ist weiter angedeutet, wie der Zylinder 17, der Luftspalt 18 und der Raum hinter und vor der Hülse 19 von der Ablagerung von Fasern, Schmutz und anderen störenden Teilen freigehalten werden können. Hierzu wird die durch den Luftspalt 18 hinter den Kolben 11 strömende Luft (Pfeile 33) derart geführt, daß sie in den Zwischenraum zwischen dem feststehenden und dem beweglichen Teil eindringt und von dort durch das Faserband mitgenommen wird. Den gleichen Weg nehmen also auch die genannten Verunreinigungen, bevor sie sich an einem der Teile festsetzen können.

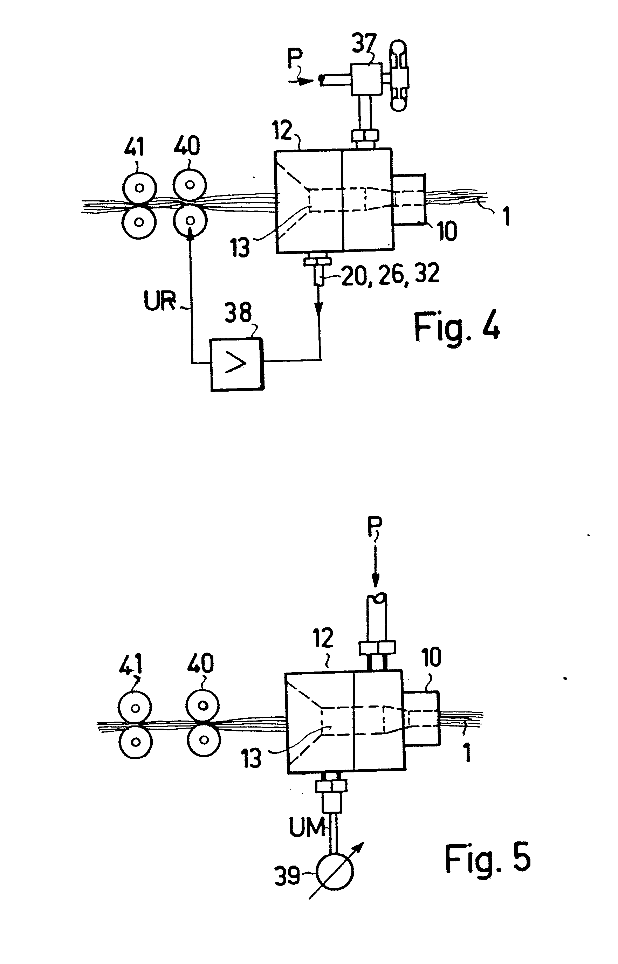

Fig. 4 und 5 zeigen den Meßtrichter als Regel- oder Meßorgan. Gemäß Fig. 4 ist die Druckluft P über ein Regelventil 37 an den Meßtrichter gelegt. Die Ausgangsgröße an den Leitungen 20, Klemmen 26 oder an der Luftaustrittsöffnung 32 wird in einem Druckwandler/Verstärker 38 in ein Regelsignal (UR) transformiert, das beispielsweise den Verzug zwischen einem hinteren Verzugswalzenpaar 41 und einem vorderen Verzugswalzenpaar 40 beeinflußt. Solange das Faserband 1 zu großen Querschnitt aufweist, beeinflußt das Regelsignal das Verzugssystem 40, 41 derart, daß der Verzug erhöht wird, während zu geringer Querschnitt den Verzug erniedrigt und damit den Faserbandquerschnitt größer werden läßt.

Fig. 5 zeigt schließlich den erfindungsgemäßen Meßtrichter als Meßorgan. In diesem Fall wird die Ausgangsgröße an den Leitungen 20 bzw. Klemmen 26 bzw. Luftaustrittsöffnungen 32 an einem geeigneten Instrument angezeigt, ohne daß auf die Größe des Verzuges im Verzugssystem 40, 41 eingewirkt wird.

**Patentansprüche**

1. Vorrichtung zur Bestimmung des Querschnitts von Faserbändern, bestehend aus einem eine durchgehende Bohrung (13) aufweisenden Meßtrichter, durch den die Faserbänder unter gleichzeitiger Verdichtung gezogen werden, sowie einer Anordnung zur Ermittlung der für den jeweiligen Faserbandquerschnitt charakteristischen Durchzugskraft, dadurch gekennzeichnet, daß der Meßtrichter aus einem festen Teil (12,17) und einem beweglichen Teil (10, 11) in Form eines Kolbens besteht, durch den sich die Bohrung (13) fortsetzt, wobei auf den beweglichen Teil (10, 11) eine Kraft ausgeübt wird, die der durch die Reibung der Faserbänder (1) in der Bohrung (13) erzeugten Mitnahmetendenz entgegenwirkt und die vom Querschnitt der Faserbänder abhängig ist, und daß eine Einrichtung (15, 16; 23, 24; 31, 32) zur Messung der Relativverschiebung zwischen dem festen und dem beweglichen Teil vorgesehen ist.

2. Vorrichtung nach Anspruch 1, dadurch gekennzeichnet, daß der feste Teil des Meßtrichters aus einem trichterförmigen Teil (12) und einem zylinderförmigen Teil (17) besteht, und daß der bewegliche Teil aus einem im zylindrischen Teil (17) axial beweglich gelagerten Organ (10) mit trichterseitig angeformtem Kolben (11) besteht.

3. Vorrichtung nach Anspruch 1 oder 2, dadurch gekennzeichnet, daß sich die Bohrung (13) im Bereich des beweglichen Teils (10, 11) längs eines Abschnitts (14) in Laufrichtung des Faserbandes (1) konisch verjüngt.

4. Vorrichtung nach Anspruch 1, dadurch gekennzeichnet, daß die auf den Kolben (11) entgegen die Mitnahmetendenz des Faserbandes (1) wirkende Kraft durch ein Druckmedium, insbesondere durch Druckluft (P) erzeugt wird.

5. Vorrichtung nach Anspruch 1, dadurch gekennzeichnet, daß die auf den Kolben (11) entgegen die Mitnahmetendenz des Faserbandes (1) wirkende Kraft mittels einer elektromagnetischen Anordnung (21) erzeugt wird.

6. Vorrichtung nach Anspruch 5, dadurch gekennzeichnet, daß die elektromagnetische Anordnung aus einer induktiv wirkenden Einheit, insbesondere einer im Zylinder (17) angeordneten Induktionsspule (21) und einem hierzu konzentrischen, am beweglichen Organ (10) befestigten Tauchmagnet (22) besteht.

7. Vorrichtung nach einem der Ansprüche 1 bis 6, dadurch gekennzeichnet, daß Abweichungen der Lage des Kolbens (11) von seiner Soll-Lage ein Meßsignal (UM) bzw. ein Regelsignal (UR) auslösen.

8. Vorrichtung nach einem oder mehreren der Ansprüche 1 bis 4, dadurch gekennzeichnet, daß der Sollwert des Faserbandquerschnittes durch die Größe des angelegten Druckes (P) einstellbar ist.

9. Vorrichtung nach den Ansprüchen 1, 5 oder 6, dadurch gekennzeichnet, daß der Sollwert des Faserbandquerschnittes durch die Größe einer an der induktiv wirkenden Einheit (21, 22) liegenden Spannung bzw. eines die sie durchfließenden Stromes einstellbar ist.

10. Vorrichtung nach Anspruch 1, dadurch gekennzeichnet, daß die sich beim Durchgang des Faserbandes einstellende Lage des Kolbens (11) mittels eines elektrischen Sensors bestimmt wird.

11. Vorrichtung nach Anspruch 10, dadurch gekennzeichnet, daß als Sensor ein auf eine Feldplatte (16) wirkendes Weicheisenteil (15) vorgesehen ist.

12. Vorrichtung nach Anspruch 10, dadurch gekennzeichnet, daß als Sensor eine aus einer Tauchspule (23) und einem Weicheisenring (24) bestehende Induktivität verwendet ist.

13. Vorrichtung nach Anspruch 1, dadurch gekennzeichnet, daß die sich beim Durchgang des Faserbandes (1) einstellende Lage des Kolbens (11) mittels eines pneumatischen Sensors bestimmt wird.

14. Vorrichtung nach Anspruch 13, dadurch gekennzeichnet, daß als pneumatischer Sensor mindestens eine die Wandung des Zylinders (17) radial durchsetzende Bohrung (31) vorgesehen ist, welche vom Kolben (11) in Abhängigkeit von seiner jeweiligen Lage zumindest teilweise abgedeckt wird, und daß an die Radialbohrung (31) Mittel zur Messung des Restdruckes (P2) angeschlossen sind.

15. Vorrichtung nach Anspruch 13 oder 14, dadurch gekennzeichnet, daß zwischen dem Kolben (11) und der Innenwand des Zylinders (17) ein Luftspalt (18) vorgesehen ist.

16. Vorrichtung nach einem oder mehreren der vorhergehenden Ansprüche, dadurch gekennzeichnet, daß an den Kolben (11) trichterseitig eine zur Bohrung (13) konzentrische Hülse (19) angeformt ist, die als Sensorträger dient.

17. Vorrichtung nach Anspruch 15, dadurch gekennzeichnet, daß die durch den Spalt (18) zwischen Kolben (11) und Zylinder (17) strömende Luft (33) in die das Faserband (1) führende Bohrung (13) rückgeführt ist.

## Claims

1. An apparatus for determining the cross-section of fibre slivers, the apparatus comprising a measuring funnel having a throughgoing bore through which the fibre slivers are drawn while being simultaneously compressed, and an arrangement for determining the force required to draw through the fibre slivers, which is a characteristic for the respective fibre sliver cross-section, characterised in that the measuring funnel comprises a fixed part (12, 17) and a movable part (10, 11) in the form of a piston through which the bore (13) is continued, wherein a force is exerted on the movable part (10, 11) which counteracts the drag tendency generated by the friction of the fibre slivers (1) in the bore (13) and which is dependent on the cross-section of the fibre slivers; and in that a device (15, 16; 23, 24; 31, 32) is provided for measuring the relative displacement between the fixed part and the movable part.

2. An apparatus in accordance with claim 1, characterised in that the fixed part of the measuring funnel comprises a funnel-like part (12) and a cylinder-like part (17); and in that the movable part comprises a member (10) axially movably journalled in the cylindrical part (17) with the piston (11) formed at the funnel side.

3. Apparatus in accordance with claim 1 or claim 2, characterised in that the bore (13) tapers conically in the direction of movement of the fibre sliver (1) over a portion (14) in the region of the movable part (10, 11).

4. Apparatus in accordance with claim 1, characterised in that the force acting on the piston (11) against the drag tendency of the fibre sliver (1) is generated by a pressure medium, in particular by compressed air (P).

5. Apparatus in accordance with claim 1, characterised in that the force acting on the piston (11) against the drag tendency of the fibre sliver (1) is generated by means of an electromagnetic arrangement (21).

6. Apparatus in accordance with claim 5, characterised in that the electromagnetic arrangement consists of an inductively operating unit, in particular an induction coil (21) arranged in the cylinder (17) and a plunger magnet (22) concentrical thereto and secured to the movable member (10).

7. Apparatus in accordance with one of the claims 1 to 6, characterised in that deviations of the position of the piston (11) from its desired position initiate a measurement signal (UM) and/or a regulating signal (UR).

8. Apparatus in accordance with one or more of the claims 1 to 4, characterised in that the the desired value for the fibre sliver cross-section is adjustable via the size of the applied pressure (P).

9. Apparatus in accordance with the claims 1, 5 or 6, characterised in that the desired value for the fibre sliver cross-section in adjustable through the size of a voltage applied to the inductively operating unit (21, 22), or by the size of a current flowing therethrough.

10. Apparatus in accordance with claim 1, characterised in that the position of the piston (11) arising due to the passage of the fibre sliver is determined by an electrical sensor.

11. Apparatus in accordance with claim 10, characterised in that a soft iron component (15) acting on a field plate (16) is provided as the sensor.

12. An apparatus in accordance with claim 10, characterised in that an inductor consisting of a plunger-type coil (23) and a soft iron ring (24) is used as the sensor.

13. Apparatus in accordance with claim 1, characterised in that the position of the piston (11) arising due to the passage of the fibre sliver (1) is determined by means of a pneumatic sensor.

14. Apparatus in accordance with claim 13, characterised in that at least one bore (31) which radially penetrates the wall of the cylinder (17) and which is at least partly covered by the piston (11) in dependence on its actual position is provided as the pneumatic sensor; and in that means for measuring the residual pressure (P2) are attached to the radial bore (31).

15. Apparatus in accordance with claim 13 or 14, characterised in that an air gap (18) is provided between the piston (11) and the inner wall of the cylinder (17).

16. Apparatus in accordance with one or more of the preceding claims, characterised in that a sleeve (19) which is concentric to the bore (13) and which acts as a carrier for the sensor is formed on the funnel side of the piston (11).

17. Apparatus in accordance with claim 15, characterised in that the air (33) flowing through the gap (18) between the piston (11) and the cylinder (17) is returned to the bore (13) guiding the fibre sliver (1).

## Revendications

1. Dispositif pour la détermination de la section transversale de rubans de fibres, constitué par un entonnoir de mesure présentant un alésage (13) de part en part, à travers lequel les rubans de fibres sont tirés avec un resserrage simultané, et par un agencement pour déceler la force de traction caractéristique de la section transversale concernée du ruban de fibres, caractérisé en ce que l'entonnoir de mesure est constitué par une partie fixe (12, 17) et une partie mobile (10, 11) sous forme d'un piston à travers lequel se prolonge l'alésage (13), tandis que sur la partie mobile (10, 11) est exercée une force qui s'oppose à la tendance à l'entraînement produite par la friction des rubans de fibres (1) dans l'alésage (13), et en ce qu'un dispositif (15, 16; 23, 24; 31, 32) pour la mesure du déplacement relatif entre les parties fixe et mobile est prévu.

2. Dispositif suivant la revendication 1, caractérisé en ce que la partie fixe de l'entonnoir de mesure est constituée par une partie en entonnoir (12) et une partie cylindrique (17), et en ce que la partie mobile est constituée par un organe (10) supporté à déplacement axial dans la partie cylindrique (17) avec un piston (11) rapporté côté entonnoir.

3. Dispositif suivant la revendication 1 ou 2, caractérisé en ce que l'alésage (13) se rétrécit coniquement dans le sens de circulation du ruban de fibres (1) au voisinage de la partie mobile (10, 11) sur une certaine longueur (14).

4. Dispositif suivant la revendication 1, caractérisé en ce que la force agissant sur le piston (11) à l'encontre de la tendance à l'entraînement du ruban de fibres (1) est produite par un agent de pression, en particulier par de l'air comprimé (P).

5. Dispositif suivant la revendication 1, caractérisé en ce que la force agissant sur le piston (11) à l'encontre de la tendance à l'entraînement du ruban de fibres (1) est produite au moyen d'un agencement électromagnétique (21).

6. Dispositif suivant la revendication 5, caractérisé en ce que l'agencement électromagnétique est constitué par une unité à action inductive, en particulier une bobine d'induction (21) agencée dans le cylindre (17) et un aimant plongeur (22) concentrique à celle-ci, fixé à l'organe mobile (10).

7. Dispositif suivant l'une des revendications 1 à 6, caractérisé en ce que des écarts de la position du piston (11) à partir de sa position nominale déclenchent un signal de mesure (UM) ou un signal de réglage (UR).

8. Dispositif suivant une ou plusieurs des revendications 1 à 4, caractérisé en ce que la valeur nominale de la section transversale du ruban de fibres peut être ajustée par la grandeur de la pression appliquée (P).

9. Dispositif suivant les revendications 1, 5 ou 6, caractérisé en ce que la valeur nominale de la section transversale du ruban de fibres peut être ajustée par la grandeur d'une tension appliquée à l'unité à action inductive (21, 22), ou d'un courant la parcourant.

10. Dispositif suivant la revendication 1 caractérisé en ce que la position du piston (11) s'établissant lors du passage du ruban de fibres est déterminée au moyen d'un capteur électrique.

11. Dispositif suivant la revendication 10, caractérisé en ce qu'on prévoit en tant que capteur, une pièce en fer doux (15) agissant sur une plaque de champ (16).

12. Dispositif suivant la revendication 10, caractérisé en ce qu'on utilise en tant que capteur, une inductance constituée par une bobine plongeante (23) et une bague en fer doux (24).

13. Dispositif suivant la revendication 1, caractérisé en ce que la position du piston (11) s'établissant lors du passage du ruban de fibres (1) est déterminée au moyen d'un capteur pneumatique.

14. Dispositif suivant la revendication 13, caractérisé en ce qu'on prévoit en tant que capteur pneumatique, au moins un alésage (31) traversant radialement la paroi du cylindre (17), qui est recouvert au moins partillement par le piston (11) en fonction de sa position concernée, et en ce qu'à l'alésage radial (31) sont raccordés des moyens pour la mesure de la pression résiduelle (P2).

15. Dispositif suivant la revendication 13 ou 14, caractérisé en ce qu'entre le piston (11) et la paroi interne du cylindre (17) est prévue une fente à air (18).

16. Dispositif suivant une ou plusieurs des revendications précédentes, caractérisé en ce que sur le piston (11) est rapportée côté entonnoir, une douille (19) concentrique à l'alésage (13), qui sert de support de capteur.

17. Dispositif suivant la revendication 15, caractérisé en ce que l'air (33) s'écoulant à travers la fente (18) entre le piston (11) et le cylindre (17), est renvoyé dans l'alésage (13) guidant le ruban de fibres (1).

Fig. 1

Fig. 2

Fig. 3

Fig. 4

Fig. 5